(19) Europäisches Patentamt
European Patent Office
Office européen des brevets

(11) Publication number: **0 068 594**
**B1**

(12) **EUROPEAN PATENT SPECIFICATION**

(45) Date of publication of patent specification: **05.06.85**

(51) Int. Cl.$^4$: **C 12 N 11/04,** C 12 P 1/00 // C12P33/06, C12P33/02

(21) Application number: **82200822.3**

(22) Date of filing: **30.06.82**

(54) **Process for carrying out an enzymatic reaction.**

(30) Priority: **01.07.81 NL 8103168**

(43) Date of publication of application: **05.01.83 Bulletin 83/01**

(45) Publication of the grant of the patent: **05.06.85 Bulletin 85/23**

(84) Designated Contracting States: **AT BE CH DE FR GB IT LI LU NL SE**

(56) References cited:
**EP-A-0 028 900**
**GB-A-1 574 269**
**US-A-4 100 029**

**RUSSIAN CHEMICAL REVIEWS, vol. 49, no. 5, May 1980, pages 385-404, Letchworth (GB); K. MARTINEK et al.: "The stabilisation of enzymes-a key factor in the practical application of biocatalysis".**

(73) Proprietor: **NEDERLANDSE CENTRALE ORGANISATIE VOOR TOEGEPAST-NATUURWETENSCHAPPELIJK ONDERZOEK**
**Juliana van Stolberglaan 148**
**NL-2595 CL The Hague (NL)**

(72) Inventor: **Ter Meulen, Berend Philippus**
**Cornelis Hendrixstraat 90**
**NL-7371 AV Loenen (Gld) (NL)**
Inventor: **Annokkee, Gustaaf Johannus**
**Keizersmantel 16**
**NL-7361 ZN Beekbergen (NL)**

(74) Representative: **van der Beek, George Frans et al**
**Nederlandsch Octrooibureau Johan de Wittlaan 15 P.O. Box 29720**
**NL-2502 LS 's-Gravenhage (NL)**

(56) References cited:
**CHEMICAL ABSTRACTS, vol. 94, no. 7, 16th February 1981, page 435, no. 45573w, Columbus Ohio (USA); J.M.C. DUARTE et al.: "The use of free and immobilized cells in the presence of organic solvents: the oxidation of cholesterol by Nocardia rhodochrous".**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

CHEMICAL ABSTRACTS, vol. 91, no. 25, 17th December 1979, page 518, no. 209350n, Columbus Ohio (USA); T. YAMANE et al.: "Steroid bioconversion in water-insoluble organic solvents: deltal-Dehydrogenation by free microbial cells and by cells entrapped in hydrophilic or lipophilic gels".

**Description**

The invention relates to a process for carrying out an enzymatic reaction in which an enzyme containing aqueous phase is contacted with a solution of a substrate in a water-immiscible solvent, and the enzyme-containing aqueous phase is used in the form of a gel.

It was known to carry out an enzymatic reaction in a biphasic system, for example from U.S. Patent Specification 3,926,726. The purpose of the use of a biphasic system is to avoid the disadvantage of the generally low solubility of the substrate and of the product in aqueous medium. According to the above-mentioned U.S. Patent Specification the enzyme is incorporated in a buffer to which, if desired, the relative coenzyme may be added as well, and this aqueous phase is dispersed in a water-immiscible solvent in which the substrate is dissolved. Substrates used are mainly steroids, but olive oil and butanol have been mentioned as substrates as well. Esters and ethers, hydrocarbons and halogenated hydrocarbons have been suggested as organic solvents.

In Biotechnology and Bioengineering XVIII, pages 815—826 (1975) the conversion of cholesterol to cholest-4-en-3-one by means of whole cells of a *Nocardia sp.* is described. In this process the substrate was dissolved in an organic, water-immiscible solvent (carbon tetrachloride), and the micro-organisms were suspended in a buffer. Although it is advantageous that the whole cells may be separated after completion of the reaction the enzyme activity is not maintained during long periods of time. In less than three days this activity falls to half the initial value after seven runs. Further disadvantages of the use of whole cells are the introduction of an additional resistance with respect to substrate exchange by the cell wall, and the low specificity of the reaction caused by the presence of a variety of enzyme systems in the whole cell. The process according to U.S. Patent Specification 3,926,726 in which the solution of the enzyme system in a buffer is dispersed in the organic solvent has the disadvantage that the enzymes become inactivated rather quickly, especially in systems requiring coenzymes.

It was also tried to limit the rapid decrease of the activity by immobilization of the enzymes. Thus, it is suggested in Biotechnology and Bioengineering XXI, pages 39—48 (1979) to couple 20β-hydroxysteroid dehydrogenase to CNBr activated Sepharose, to suspend the 20β-HSDH Sepharose particles in a phosphate buffer containing also $NAD^+$, sodium pyruvate and LDH, and to shake this suspension with a solution of testosterone in ethyl acetate. Separate immobilization of the LDH by coupling to Sepharose was suggested as well. Also, 20β-HSDH and ADH have been used. A disadvantage of this method is that it is impossible to immobilize the total enzyme system, but only part thereof, and that, consequently, the components used in the reaction, namely the coenzyme used, have to be added continuously.

Russian Chemical Reviews, vol. *49*, No. 5 (1980) pages 385—404 discusses the use of a two-phase system consisting of a substrate solution in a water-immiscible solvent and an optionally immobilised enzyme-containing aqueous phase. The two-phase system may be a suspension in the organic medium of porous particles, for example porous glass or ceramic, a hydrophilic gel, etc., impregnated by the aqueous enzyme solution. Among others, Biotechnology and Bioengineering Vol. XIX, pages 1351—1361 (1977) is mentioned as a literature reference. The latter article mentions the same immobilisation techniques together with encapsulation or entrapment in hollow fibres. The method actually disclosed in the last-mentioned article uses porous glass as a carrier for the aqueous enzyme solution.

It is also known to carry out a two-phase enzymatic reaction with *N. rhodochrous* cells entrapped in calcium alginate or polyacrylamide gels [Chem. Abstr. *94,* No. 7 (1981), page 435, No. 45573w]. Various immobilisation techniques for use in aqueous one-phase systems are discussed in European Patent application 0028900. Entrapment in calcium alginate gels is disclosed as a preferred method.

It was found that the enzymatic reaction in which an enzyme containing aqueous phase is contacted with a solution of a substrate in a water-immiscible solvent may be carried out easily and efficiently and, if desired continuously, if the enzyme containing aqueous phase is used in the form of a non-cross-linked gelatin gel.

Although it has been suggested to use hydrophilic gels generally for entrapment of enzymes, and to use the enzymes so immobilised in two-phase systems, one would not think of non-cross-linked gelatin which cannot be used in aqueous systems due to swelling or even dissolution, and has low mechanical strength. Surprisingly, non-cross-linked gelatin gels are very well suitable for use in two-phase systems. These gels have sufficient mechanical strength and, moreover show higher activity in comparison with cross-linked gelatin gels. Another advantage over cross-linked gelatin gels is that, generally, cross-linking reactions will cause loss of enzyme activity. Moreover, all of the substances present in the gel are hydrophilic, so that these will not diffuse to the organic phase even if they are not bonded chemically to the gel. The gel may contain all of the substances necessary for the enzyme system, such as coenzymes, agents for the regeneration of the coenzyme, and buffer substances. However, the agents for the regeneration of the coenzyme may be added to the organic phase as well.

Preferably, the gel is used in the form of small spheres. The preparation of the enzyme containing gel spheres may be carried out for example as follows:

The gelatin is dissolved in an amount sufficient for the gel formation, in a warm buffer solution the pH of which is in the optimum range for the enzyme system to be used. Generally, a phosphate buffer may be used, but other buffers not affecting the enzyme system, may be used. Further, substances having a stabilizing activity on the enzyme system used, such as albumin may be added. Albumin is known to be an

enzyme stabiliser (see British patent specification 1,574,269). The white of eggs, for example the white of hen's eggs may be added as well. This exerts a buffering as well as a stabilizing activity. Then the gelatin solution in the buffer is cooled to a temperature just above the gelling point of the gelatin. At this temperature the enzymes and coenzymes which may have been dissolved in a small amount of buffer, are added to the solution.

Then the whole solution is poured, with agitation, into the organic solvent to be used in the two-phase reaction, the temperature of the organic solvent being the same as that of the solution added. This operation results in a dispersion of small droplets in the organic phase. The dispersion is then cooled with agitation to a temperature below the gelling temperature, for example to 5°C, which results in the formation of gel spheres. Agitation of the spheres during some hours at low temperature hardens the spheres and will prevent sticking.

If desired, the spheres may be separated from the dispersion and frozen. The frozen spheres may be stored for long periods of time at a temperature lower than 0°C without substantial loss of enzymatic activity. After thawing, the spheres may be used again for the enzymatic reaction.

The solvent to be used in the process according to the invention should be immiscible with water or aqueous solutions under the conditions generally used for enzymatic reactions. Preferably, however, the starting materials and the reaction products should be readily soluble in the organic solvent, so as to ensure the lowest possible volume to be processed. Further, the organic solvent should be insoluble in water or have a very low solubility in water because, otherwise, there is a chance that the enzymes or enzyme systems will be denatured by the presence of the solvent in the aqueous phase. A slight amount of water may be soluble in the organic solvent. It is preferred to saturate the organic solvent with water so as to prevent extraction of water from the gelled aqueous phase. Also, the selection of the organic solvent depends on the possible inactivating influence of the solvent on the activity of the enzyme system.

Examples of suitable organic solvents are water-immiscible carboxylic alkyl esters, such as butyl acetate, ethyl acetate, amyl acetate, etc.; ethers, such as diethyl ether; hydrocarbons, such as n-hexane, cyclohexane, benzene or toluene; halogenated hydrocarbons, such as chloroform, carbon tetrachloride; water-immiscible alcohols, such as octanol-1.

The enzymatic reaction according to the invention may be carried out with a variety of substrates and enzyme systems. Of course, the application of the process makes sense only when the substrate and/or the end product has (have) a better solubility in the organic solvent than in water, because only then the advantage of a lower volume is obtained. An important field wherein the process may be used is the field of enzymatic reactions with steroids the water solubility of which is generally low. Examples of such enzymatic reactions are the reduction of cortisone to 20β-dihydrocortisone (4-pregnene-17α,20β,21-triol-3,11-dione) and the oxidation of cholesterol to cholest-4-en-3-one.

The reduction of cortisone to 20β-dihydrocortisone is described, among others, in Biotechnology and Bioengineering XVII, pages 1101—1108 (1975). Cortisone is reduced to 20β-dihydrocortisone (4-pregnene-17α,20β21-triol-3,11-dione) under the influence of the enzyme 20β-hydroxysteroid dehydrogenase (20β-HSDH). At the same time the coenzyme NADH is oxidized to $NAD^+$ (NADH=reduced form of nicotinamide adenine dinucleotide; $NAD^+$=oxidized form of nicotinamide adenine dinucleotide). The reaction proceeds according to the equation:

$$\text{[1]} \qquad \text{Cortisone}+\text{NADH}+H^+ \underset{}{\overset{20\beta\text{-HSDH}}{\rightleftharpoons}} 20\beta\text{-dihydrocortisone}+NAD^+$$

It would be rather expensive to use the necessary coenzyme (NADH) only once. This is the reason why the coenzyme is regenerated from $NAD^+$ by oxidation of ethanol to ethanal under the influence of the enzyme alcohol dehydrogenase (ADH). In this reaction the coenzyme $NAD^+$ is reduced to NADH according to the equation:

$$\text{[2]} \qquad C_2H_5OH+NAD^+ \overset{\text{ADH}}{\rightleftharpoons} CH_3CHO+NADH+H^+$$

An excess of ethanol is used to shift the equilibrium [2] to the right, and ethanol may be removed from the reaction mixture by physical means (for example by extraction with the organic solvent).

The oxidation of cholesterol to cholest-4-en-3-one proceeds under the influence of cholesterol oxidase with oxygen (air) as the oxidation agent.

An important advantage of the present process as compared with the use of an aqueous system dispersed in an organic solvent, such as described in U.S. Patent Specification 3,926,726 is the considerably longer life of the enzymes which, in accordance with the invention, are used in the form of a gelled aqueous solution. Generally, the stability of an enzyme or enzyme system dispersed in an aqueous buffer solution is in the order of a few days when it is in contact with an organic solvent. The half-life of the activity of cholesterol oxidase and of 20β-HSDH/ADH is in the order of one week. By using the process according to the invention a 10-fold increase of the life of the enzyme system may be obtained easily.

When using the gellified enzyme phase the reaction rate will be generally lower than when a dispersed enzyme phase is used, but the total producitivity of the enzyme system—defined as the total amount of product obtained per unity of enzyme—is considerably higher. The decrease of the reaction rate is caused by inhibition of transfer and limitation of diffusion of substrate and product into and from the sphere. These phenomena are highly dependent on various factors such as the dimensions and the solid content of the gel spheres, nature of the stabilization agents used and nature and molecular dimensions of substrate and product.

The higher total productivity of the present enzyme system, in spite of the decreased reaction rate, is due to the considerably longer life of the enzyme system. For example, with the system 20β-HSDH/ADH an about eight times higher product yield may be obtained with the process according to the invention than with the same amount of enzyme dispersed in the form of an aqueous solution in the organic solvent.

As far as the regeneration of the coenzyme used is concerned the effectivity thereof may be expressed as the maximum number of regeneration cycles, that is to say the number of times the oxidised coenzyme may be converted to the reduced form and back to the oxidized form. (For example, a maximum number of regeneration cycles of 50 corresponds with an effectively/yield per regeneration cycle of ±95%).

For the system 20β-HSDH/ADH with $NAD^+$ and NADH as the coenzyme an increase of the maximum number of regeneration cycles by a factor 10 was found when using the gel spheres.

As compared with the use of a non-gellified aqueous phase, for example according to U.S. Patent Specification 3,926,726, the present process has the advantage that separation of the organic phase from the aqueous phase does not involve specific problems. When aqueous solutions are dispersed in organic solvents it may be difficult to separate the dispersions obtained, especially when proteins or other substances of high molecular weight are present. Regeneration of the enzyme phase then may be difficult in practice. These difficulties do not occur when an aqueous phase in the form of a non-cross-linked gelatin gel is used.

Further, an important advantage as compared with the use of a non-gellified aqueous phase is that the process may be carried out easily in a continuous way, for example in a packed bed reactor.

Example I

Reduction of cortisone to 20β-dihydrocortisone

A gellified enzyme phase was prepared as follows:

Twenty % gelatin (Bloom number 60) was added to 5 ml of egg-white. This mixture was heated to a temperature of 40—60°C until the gelatin had dissolved. Then the mixture was cooled to a temperature of about 35°C in a few minutes. At this temperature the following was added to the egg-white/gelatin mixture:

— 15 µl of a 20β-HSDH suspension (about 2.5 units),
— 0.5 mg of ADH (about 250 units) and
— 5 mg of NAD.

Then the entire solution was poured into butyl acetate having a temperature of about 35°C, with stirring. The stirring was continued and the mixture was cooled as rapidly as possible (in a few minutes) to a temperature of 5°C; the mixture was stirred during about two hours at the same temperature. A dispersion of non-sticky gel spheres having a diameter of about 1 mm was obtained.

The gellified enzyme phase obtained was then added to 50 ml of organic phase prepared by dissolving 50 mg of cortisone and 0.5 ml of ethanol (96%) in 50 ml of butyl acetate saturated (about 10 ml/l) with water. The spheres where maintained in suspension by agitation at a temperature of 5°C.

The organic phase was replenished every 24 hours and the conversion obtained in 24 hours was determined by analysis of the components of the organic phase removed. The analysis was effected by thin layer chromatography and high performance liquid chromatography (HPLC). The results are given in the attached Figure 1 [curve (1)].

In the above-mentioned way also gelatin spheres were prepared starting from 5 ml of 0.05 molar $KH_2PO_4/Na_2HPO_4$ buffer (pH 7) (instead of egg-white) to which 20% of gelatin had been added. These enzyme spheres were also tested in the above-mentioned way. The results are given in Figure 1 [curve (2)].

For comparison an enzyme phase was prepared by dispersing the same amounts of HSDH, ADH, NAD as above with 7.5 mg of albumin in 5 ml of 0.05 molar $KH_2PO_4/Na_2HPO_4$ buffer (pH 7). This enzyme phase was dispersed in 50 ml of the same organic phase. In this test the organic phase was also replenished every 24 hours.

The results are also given in Figure 1 [curve (3)]. The results show that a considerable increase of the life is obtained with the process according to the invention.

The above-described reaction according to the invention was also carried out in a completely continuous way in a so-called packed bed reactor.

For this purpose 50 g (about 50 ml) of gelatin spheres having the following total composition were prepared in the above-described way:

— 10 g of gelatin
— 40 g of egg-white neutralized to pH 7 with lactic acid

—75 µl of an HSDH suspension (about 12.5 units)
—25 mg of ADH
—5 mg of NAD

A column having a height of about 15 cm and a diameter of about 2.5 cm was filled with these spheres. A solution of 0.5 g/l of cortisone and 10 ml/l of ethanol in butyl acetate was passed continuously through the so-obtained packed bed reactor at a rate of 3 ml/hour. The conversion in the reactor was determined every day by analysis of the components of the product stream.

The results are given in Figure 2. The results show that the reactor has been active during 130 days, in which period the conversion decreased gradually from 100% to 0%. In this way a total amount of 2320 mg of cortisone was converted which conversion needed only 5 mg of $NAD^+$ (coenzyme). Consequently, as far as the used $NAD^+$ is concerned, a total number of 860 regeneration cycles was reached.

Example II
Oxidation of cholesterol to cholest-4-en-5-one

Gelatin spheres containing egg-white were prepared in accordance with the procedure described in Example I.

The following enzymes were added to 5 g of spheres.

—2.5 units of cholesterol oxidase
—1000 units of catalase.

The gellified enzyme phase was added to an organic phase consisting of a solution of 50 mg of cholesterol in 50 ml of toluene, and was kept in suspension by agitation at a temperature of 5°C. Oxygen was continuously passed through the suspension via a capillary.

The organic phase was replenished every 24 hours and the conversion obtained in 24 hours was determined by thin layer chromatography and analysis of the components of the organic phase removed. The results are given in Figure 3.

For comparison an enzyme phase was prepared by dispersing the same amounts of cholesterol oxidase and catalase in 5 ml of 0.05 molar $KH_2PO_4/Na_2HPO_4$ buffer (pH 7). This enzyme phase was dispersed in 50 ml of the same organic phase. In this test the organic phase was also replenished every 24 hours. The results are stated in Figure 3 as well.

The results show that a considerable increase of the life is obtained with the gellified system.

Example III
Oxidation of testosterone to androst-4-ene-3,17-dione

$$\text{testosterone}+NAD^+ \xrightarrow{\text{17-HSDH}} \text{androst-4-ene-3,17-dione}+NADH+H^+ \quad \text{ethanal}+NADH+H^+ \xrightarrow{\text{ADH}} \text{ethanol}+NAD^+$$

---

$$\text{testosteron}+\text{ethanal} \xrightarrow[\text{ADH}]{\text{17-HSDH}} \text{androst-4-ene-3,17-dione}+\text{ethanol}$$

Gelatin spheres containing egg-white were prepared according to the procedure described in Example I.

The following enzymes were added per 2.5 g of spheres:

—5 mg of 17-HSDH (about 12 units)
—5 mg of ADH (about 2000 units)
—5 mg of $NAD^+$

The gellified phase was added to an organic phase consisting of 22.5 ml of a solution of testosterone in water-saturated butyl acetate (0.5 g of testosterone per liter of butyl acetate). Also 250 µl of ethanal were added to the organic phase. The gellified enzyme phase was kept in suspension by agitation at a temperature of 5°C. The organic phase was replenished every 24 hours and the conversion obtained in 24 hours was determined by thin layer chromatography and analysis of the components of the organic phase removed. The results are given in Figure 4 curve (1).

For comparison an enzyme phase was prepared by dispersing the same amounts of 17-HSDH, ADH and $NAD^+$ in 2.5 ml of 0.05 molar $KH_2PO_4/Na_2HPO_4$ buffer (pH 7). The enzyme phase was dispersed in 22.5 ml of the same organic phase. The organic phase was replenished every 24 hours in this test as well. The results are given in Figure 4 [curve (2)].

The results show that the gellified system has a considerably longer life and, consequently, a higher productivity than the non-gellified system.

Example IV
Storage tests with a gelatin based enzyme preparation

The enzyme preparation described in Example I and based on gelatin with egg-white was subjected to a storage test at various temperatures. The activity of the preparation after storage during a certain period of time and at a certain temperature was determined by measuring, according to Example I, the conversion of cortisone to dihydrocortisone in a batch-wise test after 24 hours.

The results of these tests are given in Figure 5. Curve (1) relates to the change of the activity with time, expressed as percentage of the initial activity, after storage at −10°C; curve (2) represents the same at +5°C, and curve (3) represents the same at +20°C. This shows that a preparation according to the invention can be stored during a long period of time (more than one year) and, consequently, may be reused.

**Claims**

1. A process for carrying out an enzymatic reaction in which an enzyme containing aqueous phase is contacted with a solution of a substrate in a water-immiscible solvent, and the enzyme containing aqueous phase is used in the form of a gel, characterized in that the gel is a non-cross-linked gelatin gel.
2. The process of claim 1, characterized in that the gel has the form of spheres.
3. The process of claim 1 or 2, characterized in that the gellified aqueous phase contains, in addition to the enzyme, also the corresponding coenzyme.
4. The process of any of claims 1—3, characterized in that the gellified aqueous phase contains a complete enzyme system.
5. The process of any of claims 1—4, characterized in that the water-immiscible phase contains agents for the regeneration of the coenzyme.
6. The process of any of claims 1—4, characterized in that the aqueous phase contains a stabilizer for the enzyme or for the enzyme system.
7. The process of claim 6, characterized in that the stabilizer is albumin.
8. The process of claim 6, characterized in that the stabilizer is egg-white.
9. The process of claim 8, characterized in that the stabilizer is the white of hen's eggs.

**Patentansprüche**

1. Verfahren zur Durchführung einer enzymatischen Reaktion, bei welcher eine Enzym enthaltende wässrige Phase mit einer Lösung eines Substrates in einem mit Wasser nicht mischbaren Lösungsmittel in Kontakt gebracht wird und die Enzym enthaltende wässrige Phase in Form eines Gels verwendet wird, dadurch gekennzeichnet, dass das Gel ein nichtvernetztes Gelatinegel ist.
2. Verfahren nach Anspruch 1, dadurch gekennzeichnet, dass das Gel die Form von Kugeln hat.
3. Verfahren nach Anspruch 1 oder 2, dadurch gekennzeichnet, dass die gelierte wässrige Phase zusätzlich zum Enzym auch das entsprechende Coenzym enthält.
4. Verfahren nach einem der Ansprüche 1 bis 3, dadurch gekennzeichnet, dass die gelierte wässrige Phase ein vollständiges Enzymsystem enthält.
5. Verfahren nach einem der Ansprüche 1 bis 4, dadurch gekennzeichnet, dass die mit Wasser mischbare Phase Mittel für die Regeneration des Coenzyms enthält.
6. Verfahren nach einem der Ansprüche 1 bis 4, dadurch gekennzeichnet, dass die wässrige Phase einen Stabilisator für das Enzym oder für das Enzymsystem enthält.
7. Verfahren nach Anspruch 6, dadurch gekennzeichnet, dass der Stabilisator Albumin ist.
8. Verfahren nach Anspruch 6, dadurch gekennzeichnet, dass der Stabilisator Eiweiss ist.
9. Verfahren nach Anspruch 8, dadurch gekennzeichnet, dass der Stabilisator Eiweiss von Hühnereiern ist.

**Revendications**

1. Procédé de mise en oeuvre d'une réaction enzymatique dans lequel une phase aqueuse contenant une enzyme est mise en contact avec une solution d'un substrat dans un solvant non miscible à l'eau, et la phase aqueuse contenant l'enzyme est utilisée sous la forme d'un gel, caractérisé par le fait que le gel est un gel de gélatine non réticulée.
2. Procédé selon la revendication 1, caractérisé par le fait que le gel a la forme de sphères.
3. Procédé selon l'une des revendications 1 ou 2, caractérisé par le fait que la phase aqueuse gélifiée contient, en plus de l'enzyme, également la co-enzyme correspondante.
4. Procédé selon l'une des revendications 1 à 3, caractérisé par le fait que la phase aqueuse gélifiée contient un système enzymatique complet.
5. Procédé selon l'une des revendications 1 à 4, caractérisé par le fait que la phase non miscible à l'eau contient des agents pour la régénération de la co-enzyme.

6. Procédé selon l'une des revendications 1 à 4, caractérisé par le fait que la phase aqueuse contient un stabilisant pour l'enzyme ou pour le système enzymatique.

7. Procédé selon la revendication 6, caractérisé par le fait que le stabilisant est l'albumine.

8. Procédé selon la revendication 6, caractérisé par le fait que le stabilisant est le blanc d'oeuf.

9. Procédé selon la revendication 8, caractérisé par le fait que le stabilisant est le blanc d'oeuf de poule.

Fig-1

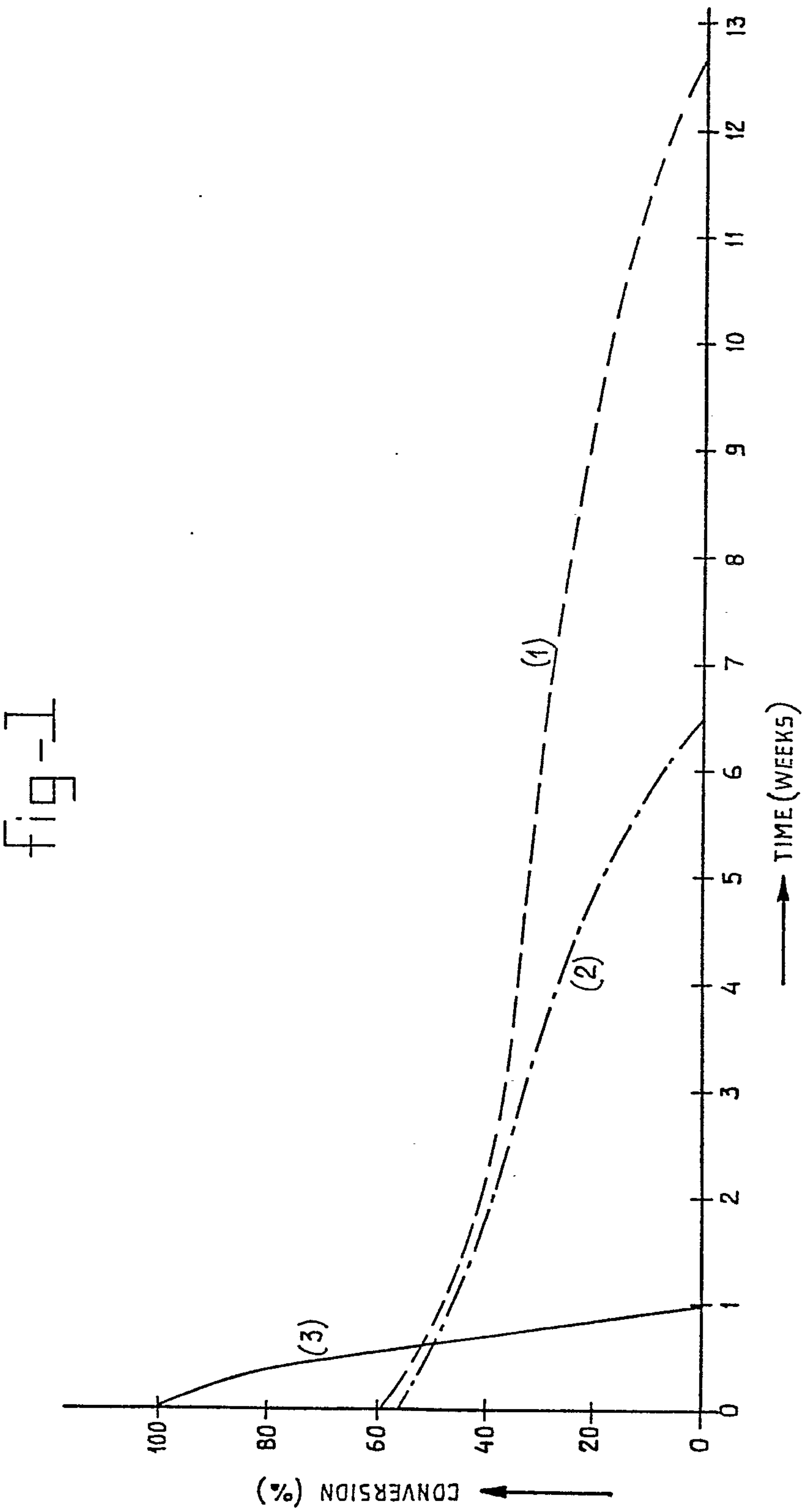
CONVERSION (%)
100
80
60
40
20
0
TIME (WEEKS)
0
1
2
3
4
5
6
7
8
9
10
11
12
13
(1)
(2)
(3)

fig-2

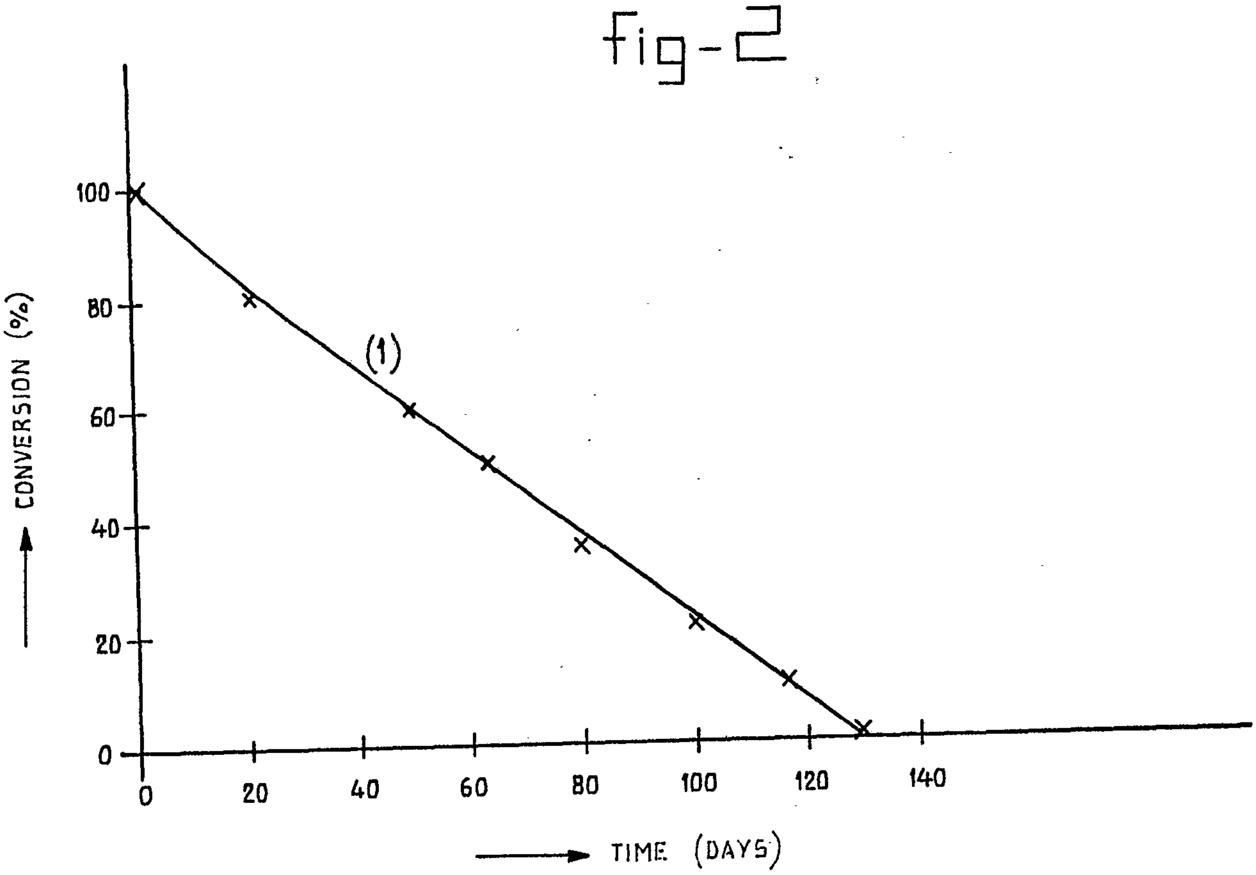

Fig-3

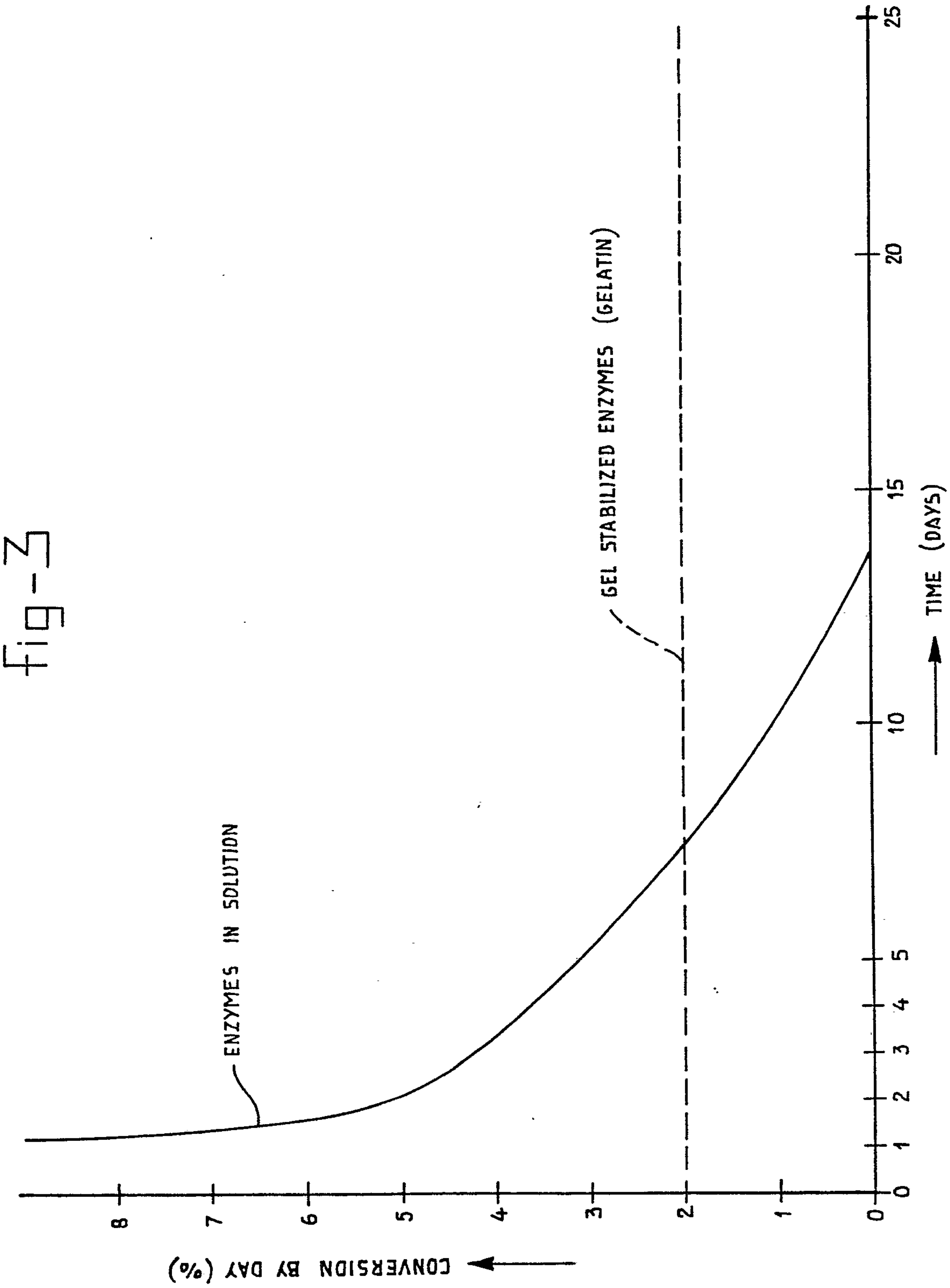

CONVERSION BY DAY (%)
0
1
2
3
4
5
6
7
8
TIME (DAYS)
0
1
2
3
4
5
10
15
20
25
ENZYMES IN SOLUTION
GEL STABILIZED ENZYMES (GELATIN)

Fig-4

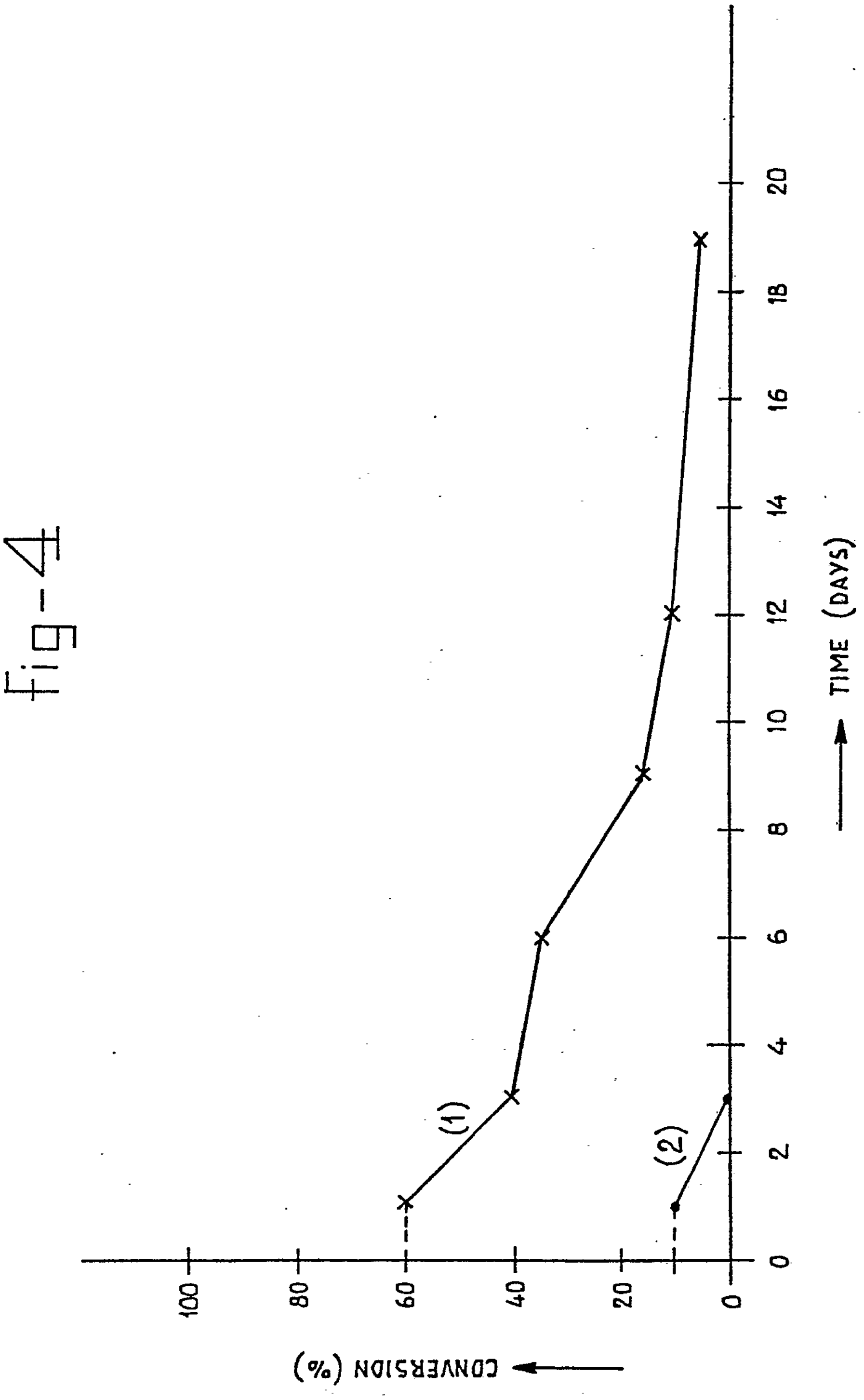

fig-5

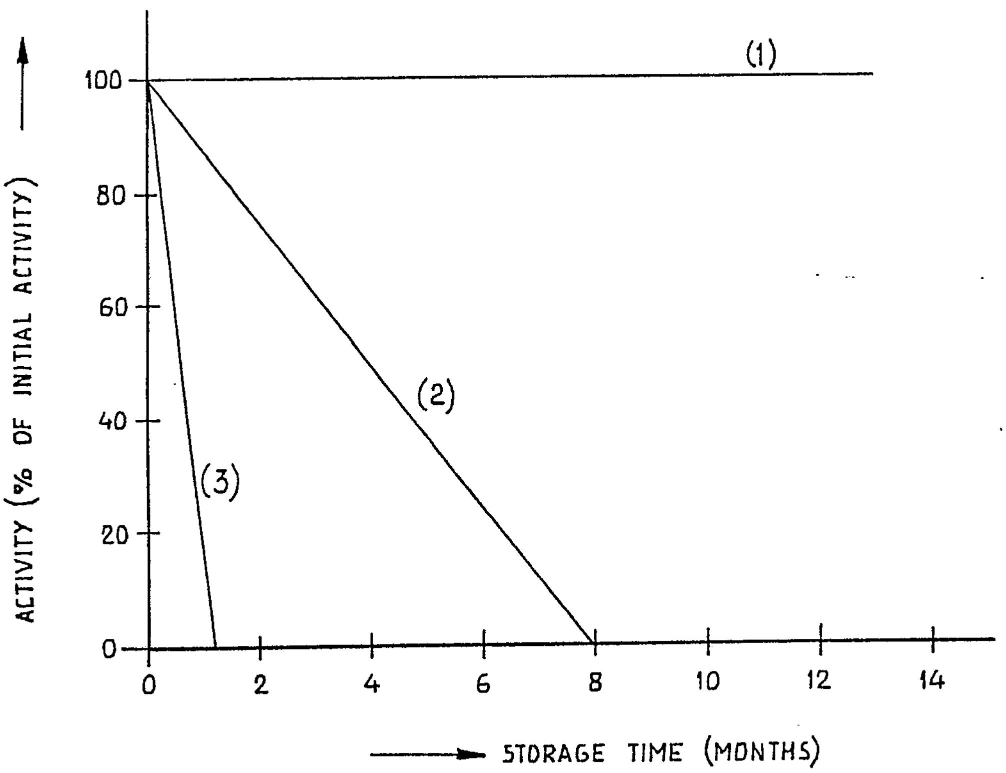
ACTIVITY (% OF INITIAL ACTIVITY)
100
80
60
40
20
0
0
2
4
6
8
10
12
14
STORAGE TIME (MONTHS)
(1)
(2)
(3)